# EUROPÄISCHE PATENTSCHRIFT

(11) **EP 2 134 669 B1**
(45) Veröffentlichungstag und Bekanntmachung des Hinweises auf die Patenterteilung: **09.01.2013**
(21) Anmeldenummer: 08709204.5
(22) Anmeldetag: 25.02.2008
(51) Int. Cl.: C07C 29/141, C07C 45/75, C07C 45/82, C07C 47/19

(54) **VERFAHREN ZUR HERSTELLUNG VON HYDROXYPIVALINALDEHYD UND NEOPENTYLGLYKOL**
METHOD FOR PRODUCING HYDROXY PIVALIN ALDEHYDE AND NEOPENTYL GLYCOL
PROCEDE DE PRODUCTION D'HYDROXYPIVALINALDEHYDE ET DE NEOPENTYLGLYCOL

(30) Priorität: 02.03.2007 EP 07103432
(43) Veröffentlichungstag der Anmeldung: 23.12.2009
(73) Patentinhaber: BASF SE, 67056 Ludwigshafen (DE)
(72) Erfinder: SIRCH, Tilman, 67105 Schifferstadt (DE); STEININGER, Michael, 67435 Neustadt (DE); MAAS, Steffen, 55270 Bubenheim (DE); RITTINGER, Stefan, 68199 Mannheim (DE); SCHLITTER, Stephan, Shanghai 200336 (CN)
(86) Internationale Anmeldenummer: PCT/EP2008/052240
(87) Internationale Veröffentlichungsnummer: WO 2008/107333

(56) Entgegenhaltungen:
- EP-A- 0 522 368
- EP-A- 1 568 678
- WO-A-98/17614
- DE-A1- 10 317 545

## Beschreibung

Die Erfindung betrifft ein Verfahren zur Herstellung von Hydroxypivalinaldehyd durch Aldolisierung von Isobutyraldehyd mit Formaldehyd sowie ein Verfahren zur Herstellung von Neopentylglykol durch Hydrierung des so erhaltenen Isobutyraldehyds.

Die Aldolisierung von Isobutyraldehyd mit Formaldehyd und die weitere Umsetzung zu Neopentylglykol sind an sich bekannt. Die WO 98/17614 betrifft beispielsweise ein Verfahren zur ununterbrochenen Herstellung von Neopentylglykol unter Verwendung von Formaldehyd, das eine Methanolkonzentration von 0,1 bis 15% aufweist. Der Austrag der Aldolisierung von Isobutyraldehyd mit wässriger Formaldehydlösung wird unter Zuhilfenahme von Octanol als Extraktionsmittel in eine wertprodukthaltige organische Phase und eine wässrige Phase aufgetrennt. Die organische Phase wird anschließend leicht andestilliert, um restliche Leichtsieder abzutrennen. Der Sumpf dieser ersten Kolonne wird hydriert, und Neopentylglykol wird als Wertprodukt nach einer weiteren Extraktion und weiterer Destillation erhalten.

Neopentylglykol wird beispielsweise mit Hydroxypivalinsäure zu Hydroxypivalinsäure-Neopentylglykolester (HPN) umgesetzt. Ein derartiges Verfahren ist beispielsweise in der EP-A-0 895 982 beschrieben.

Aufgabe der vorliegenden Erfindung ist die Bereitstellung eines Verfahrens zur Herstellung von Hydroxypivalinaldehyd durch Aldolisierung von Isobutyraldehyd mit Formaldehyd, das zu einer möglichst geringen thermischen Belastung des Produkts führt und mit geringem Aufwand eine nahezu vollständige Abtrennung des Aldehyds aus der wässrigen Lösung erlaubt.

Die Aufgabe wird erfindungsgemäß gelöst durch ein Verfahren zur Herstellung von Hydroxypivalinaldehyd durch Aldolisierung von lsobutyraldehyd mit Formaldehyd und anschließender destillativer Aufarbeitung des erhaltenen Reaktionsaustrags, bei dem der Reaktionsaustrag einer Destillationskolonne zugeführt wird, die bei einem Kopfdruck im Bereich von 0,5 bis 1,5 bar betrieben wird und in der im Kopfbereich eine zweistufige Kondensation vorgesehen ist, bei der die Brüden zunächst in einen bei einer Temperatur im Bereich von 50 bis 80°C betriebenen Partialkondensator geführt werden, dessen Kondensat zumindest teilweise in die Destillationskolonne zurückgeführt wird, und bei der die im Partialkondensator nicht kondensierten Brüden einem nachgeschalteten, bei einer Temperatur im Bereich von -40 bis +30°C betriebenen Kondensator zugeführt werden, dessen Kondensat zumindest teilweise ausgeschleust wird.

Es wurde efindungsgemäß gefunden, dass die Abscheidung der Restbrüden bei der Destillation vorteilhaft durch eine Kombination eines Partialkondensators mit nachgeschaltetem kaltem Kondensator erreicht wird. Es wurde erfindungsgemäß gefunden, dass ein Totalkondensator, der direkt nach der Kolonne betrieben wird, durch Hydroxypivalinaldehyd zugesetzt werden kann. Der Kondensator muss jedoch, wenn er die Brüden vollständig kondensieren soll, möglichst kalt betrieben werden. Der Hydroxypivalinaldehyd wird dabei aufgrund einer hohen kalten Rücklaufmenge in der Kolonne niedergeschlagen, was zu einer hohen thermischen Belastung im Sumpf der Kolonne führt, da der Verdampfer diese Energie aufbringen muss. Zudem kann der Kondensator durch Hydroxypivalinaldehyd zugesetzt werden.

Die erfindungsgemäße Fahrweise verhindert den Niederschlag von Hydroxypivalinaldehyd im Kondensator.

Zudem wird in einem Druck- und Temperaturbereich der Kolonne gearbeitet, der es erlaubt, Hydroxypivalinaldehyd in flüssiger Phase zu halten und gleichzeitig die thermische Belastung gering zu halten.

Nach der Aldolisierung werden nicht umgesetzte Aldehyde und ein Teil der vorzugsweise im Verfahren mitverwendeten Amin-Base destillativ abgetrennt und vorzugsweise zurückgeführt. Im Destillationssumpf bleiben die Produkte der Aldolisierung (Hydroxypivalinaldehyd), Wasser, Ammoniumformiat aus der Amin-Base und Ameisensäure zurück. Die destillative Abtrennung sollte bevorzugt bei moderatem Druck erfolgen, um nicht durch erhöhte Temperatur Hydroxypivalinaldehyd in Hydroxypivalinsäure-Neopentylglykolester (HPN) zu zersetzen. Andererseits sollte der Druck nicht zu gering sein, um noch am Kopf die Leichtsieder Isobutyraldehyd und Amin-Base, beispielsweise Trialkylamin wie Trimethylamin, zu kondensieren.

Die Destillation darf nicht bei zu niedrigem Druck stattfinden, da unterhalb von etwa 60°C die Löslichkeit von Hydroxypivalinaldehyd (HPA) in der wässrigen Lösung schlagartig auf etwa 1 bis 3 Gew.-% abfällt, in Abhängigkeit von Isobutyraldehyd- und Methanol-Gehalt. Methanol wird dabei über die wässrige Formaldehyd-Lösung eingeschleppt, die je nach Herstellungsbedingungen etwa 1 bis 3 Gew.-% Methanol enthält.

Wird der nicht umgesetzte Aldehyd von der wässrigen Lösung abgetrennt, so würde bei einer einstufigen Destillation Methanol mit abgetrennt, das über eine Rückführung in die Aldolisierung in den Prozess zurückgeführt würde und nur über einen weiteren Schritt, beispielsweise die Ausschleusung von Aldehyd mit Methanol, ausgeschleust werden könnte.

Erfindungsgemäß wurde gefunden, dass die vorstehend beschriebenen Nachteile mit der mehrstufigen Kondensation mit Rücklauf vermieden werden können, wobei bei der Destillation spezifische Bedingungen einzuhalten sind. Im Ergebnis wird u. a. Methanol ausreichend im Sumpf zurückgehalten.

Es hat sich gezeigt, dass bei Ausführung der destillativen Trennung nahe Umgebungsdruck, d. h. bei einem Kopfdruck im Bereich von 0,5 bis 1,5 bar absolut, am Kopf die Leichtsieder mit Wasser als Kühlmedium kondensiert werden können. Die sich einstellenden Sumpftemperaturen führen bei der erfindungsgemäßen Vorgehensweise noch zu keiner signifikanten Hydroxypivalinaldehyd-Zersetzung.

Vorzugsweise wird das Kondensat des Partialkondensators zu mehr als 70 Gew.-%, besonders bevorzugt vollständig in die Destillationskolonne zurückgeführt. Dabei wird das Kondensat vorzugsweise in den Kolonnenkopf zurückgeführt. Das Kondensat des nachgeschalteten Kondensators wird vorzugsweise zu mindestens 70 Gew.-%, insbesondere vollständig ausgeschleust.

Der Partialkondensator wird bei einer Temperatur im Bereich von 50 bis 80°C, vorzugsweise 55 bis 60°C betrieben. Der nachgeschaltete Kondensator wird bei einer Temperatur im Bereich von -40 bis +30°C, vorzugsweise -10 bis +10°C betrieben. Der Kopfdruck beträgt besonders bevorzugt 1 bis 1,2 bar.

Der Sumpf der Destillationskolonne ist bevorzugt mit einem Verdampfer mit kurzer Verweilzeit verbunden, der bei einer Temperatur im Bereich von 90 bis 130°C, besonders bevorzugt von 100 bis 105°C betrieben wird. Dabei handelt es sich bei dem Verdampfer besonders bevorzugt um einen Fallfilmverdampfer, ferner können bevorzugt ein Wischfilmverdampfer oder ein Kurzwegverdampfer eingesetzt werden. Wesentlich ist dabei, dass eine kurze Verweilzeit und damit eine geringe thermische Belastung erreicht wird. Der Verdampfer kann in geeigneter Weise mit Wärme versorgt werden, beispielsweise mit 4 bar Dampf.

Vorzugsweise wird ein mit Hydroxypivalinaldehyd angereichertes Gemisch aus dem Sumpf des Verdampfers ausgeschleust. Auch ein Ausschleusen aus dem Umlauf ist erfindungsgemäß möglich. Dieses Gemisch kann zur Verminderung der thermischen Belastung vor der weiteren Aufarbeitung in einem Kühler mit einer Kühlertemperatur im Bereich von 50 bis 80°C, besonders bevorzugt 55 bis 60°C, abgekühlt werden. Das abgekühlte Gemisch kann einem Abscheider und nachfolgend einer Hydrierung zugeführt werden. Zur Herstellung von Neopentylglykol wird zunächst wie vorstehend aldolisiert und aufgearbeitet, und nachfolgend wird eine Hydrierung des so erhaltenen Hydroxypivalinaldehyds durchgeführt.

Bevorzugt weist die Destillationskolonne Einbauten zur Erhöhung der Trennleistung auf. Dabei wird der Reaktionsaustausch der Aldolisierung vorzugsweise in einem räumlichen Bereich zwischen 1/4 und ¾ der theoretischen Böden der Destillationskolonne zugeführt, besonders bevorzugt in einem räumlichen Bereich zwischen 1/3 und 2/3 der theoretischen Böden der Destillationskolonne. Beispielsweise kann die Zuführung etwas oberhalb der Mitte der theoretischen Böden erfolgen (Verhältnis 3:4).

Die destillativen Einbauten können beispielsweise als geordnete Packung vorliegen, beispielsweise als Blechpackung wie Mellapak 250 Y oder Montz Pak, Typ B 1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckveriust und der geringe spezifische Flüssig-Hold-up im Vergleich zu beispielsweise Ventilböden.

Im Partialkondensator fällt als Kondensat überwiegend Wasser an, das vorzugsweise der Kolonne vollständig als Rücklauf zugeführt wird. Beispielsweise kann ein Gemisch als Kondensat erhalten werden, das etwa 10 Gew.-% Isobutyraldehyd, etwa 5 Gew.-% Amin-Base wie Trimethylamin, etwa 1 Gew.-% Hydroxypivalinaldehyd und etwa 5 Gew.-% Methanol neben Wasser enthält. Die Restbrüden enthalten die überwiegende Menge an Isobutyraldehyd und Amin-Base wie Trimethylamin. Diese werden in dem nachgeschalteten Kondensator möglichst vollständig niedergeschlagen. Als Kühlmedium kann hierbei bevorzugt möglichst kaltes Wasser (z. B. etwa 5°C) oder eine Kältemischung (z. B. Glykol-Wasser mit beispielsweise -20°C) verwendet werden.

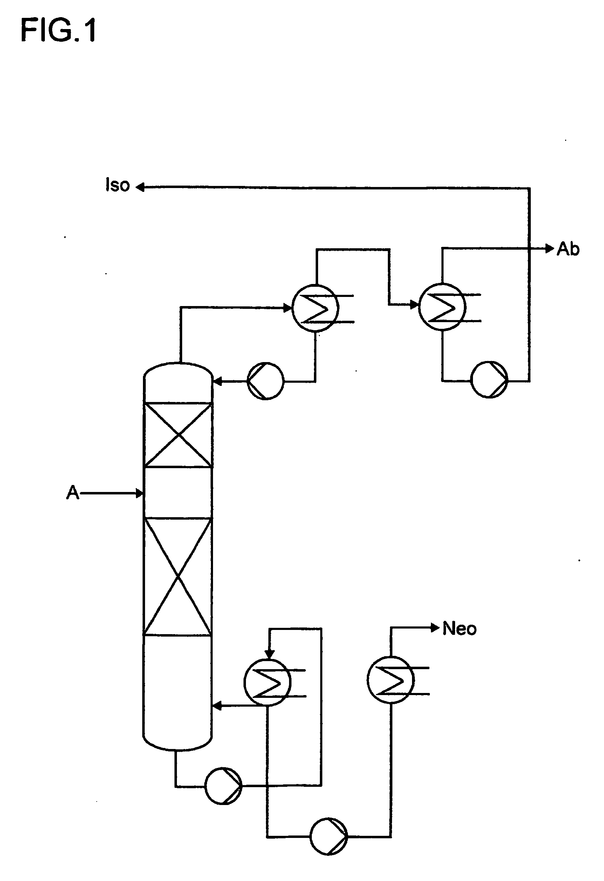

In der beigefügten Zeichnung ist die destillative Aufarbeitung des Aldolisierungs-Reaktionsproduktes schematisch dargestellt. Der Aldolisierungsaustrag (A) wird an der gezeigten Stelle in der Mitte der Destillationskolonne zugeführt. Am Kolonnenkopf schließt sich eine zweistufige Kondensation an, wobei letztendlich ein Abgas (Ab) abgeführt wird. Im Kolonnensumpf wird Isobutyraldehyd zurückgeführt und verdampft. Ausgeschleuster Hydroxypivalinaldehyd wird zur Neopentylglykolhydrierung (NEO) weitergeleitet. Aus der Kondensation wird Isobutyraldehyd (ISO) zurückgeführt.

Die Erfindung wird durch die nachstehenden Beispiele näher erläutert.

### Beispiele

### Aldolisierung von Isobutyraldehyd (IBA) mit Formaldehyd

Es wurden ca. 750 g/h Isobutyraldehyd (ca. > 99,5 GC-Flächen% IBA) mit ca. 700 g/h Formaldehyd (ca. 49% Formaldehyd, 1,5% Methanol, Rest Wasser) und 80 g/h Trimethylaminlösung (50% TMA in Wasser) in einer zweistufigen Rührkesselkaskade zur Reaktion gebracht.

### IBA-Rückführung Beispiel 1

Anschließend wurde die Lösung in einer Kolonne destillativ von Leichtsiedern befreit. Die Kolonne (Durchmesser 30 mm) ist mit 2 m Gewebepackung (500 m²/m³ spezifische Oberfläche) im Verstärkungsteil und 4 m Blechpackung (250 m²/m³) ausgerüstet. Der Aldolisierungsaustrag wurde oberhalb der Blechpackung zugeführt, am Kopf der Kolonne wurde das Destillat gasförmig bei ca. 85°C abgezogen und einem Partialkondensator zugeführt. Dort wurde es mittels Wasser bei 55°C abgekühlt. Das hierbei anfallende Kondensat (ca. 50 g/h) wurde vollständig der Kolonne zugeführt, die Restbrüden wurden dem Nachkondensator zugeführt. Dort wurden diese mittels einer Glykol-Wasser-Kältemischung bei -20°C nahezu vollständig kondensiert. Das erhaltene Kondensat (von ca. 80 g/h) wurde dem ersten Rührkessel zugeführt. In der dem Kondensator nachgeschalteten Kühlfalle fielen ca. 1 g/h Flüssigkeit an (ca. 80% IBA, ca. 20% TMA).

Die IBA-Abtrennung wurde bei einem Kopfdruck von ca. 1 bar absolut betrieben. Als Verdampfer wurde ein Fallfilmverdampfer verwendet. Es wurde eine Sumpftemperatur im Sumpf der Kolonne von 102°C eingestellt. Die Rücklaufmenge (bzw. Kühlwassermenge des Partialkondensators) zur Kolonne wurde mittels der Temperatur in der Mitte der Gewebepackung geregelt, es wurde eine Temperatur von 85°C eingestellt.

Aus dem Kolonnensumpf wurde mittels einer Pumpe ca. 100 kg/h Flüssigkeit abgezogen. Diese wurde dem Fallfilmverdampfer (bestehend aus einem ölbeheizten Edelstahlrohr, Länge, 2,5 m, Innendurchmesser ca. 21 mm, Wandstärke ca. 2 mm) zugeführt. Es wurde aus dem Sumpf des Fallfilmverdampfers ca. 1,5 kg/h Produkt mit einer Konzentration von ca. 0,3% Isobutyraldehyd abgezogen. Die Brüden und überschüssige Flüssigkeit wurden dem Kolonnensumpf zugeführt. Das ausgeschleuste Sumpfprodukt enthielt ca. 70% HPA, ca. 1,5% HPN, 0,3% IBA, Rest Wasser.

### Hydrierung von Hydroxypivalinaldehyd zu Neopentylglykol

### Katalysatoraktivierung

150 ml eines Cu/Al₂O₃-Katalysators wie in der EP 44444 beschrieben wurden in einem Rohrreaktor bei 190°C durch Überleiten eines Gemisches aus 5 Vol.% Wasserstoff und 95 Vol.% Stickstoff (Gesamtvolumen 50 NI/h) drucklos für 24 h aktiviert.

### Hydrierung

Als Ausgangslösung diente das vorstehend als Hydrierfeed beschriebene Gemisch. Dem Gemisch wurden ca. 10 Gew.-% bezogen auf den Hydrierfeed einer 15%-igen wässrigen Lösung von Trimethylamin zugesetzt. Der so erhaltene Zulauf wurde in Rieselfahrweise bei 40 bar H₂-Druck über den auf 120°C beheizten Reaktor gefahren. Die Belastung betrug 0,4 kg HPA (I_{Kat.}*h). Ein Teil des Hydrieraustrags wurde dem Zulauf wieder zugemischt (Kreislauffahrweise). Das Verhältnis von Kreislauf zu Zulauf betrug 10:1. Der pH-Wert wurde von Proben des Reaktoraustrags bei Raumtemperatur zu 8,9 gemessen.

Es wurde eine wässrige Lösung mit ca. 69% NPG, ca. 1,8% HPN, ca. 2 % Isobutanol, ca. 3,5% Methanol, ca. 2% TMA, Rest Wasser nach der Hydrierung erhalten.

### IBA-Rückführung Beispiel 2

Bei sonst gleichen Bedingungen der Aldolisierung wurde am Kopf der IBA-Rückführung ein Kondensator mit Kühlwasser (ca. 10°C) und einem nachgeschalteten Phasenscheider verwendet. Die Sumpftemperatur der Kolonne wurde auf 102°C eingestellt. Am Kopf wurde das Destillat gasförmig dem Kondensator zugeführt. Es fielen ca. 255 g/h flüssiges Kondensat an. In dem nachgeschalteten Phasenscheider wurde eine wässrige Phase von 95 g/h abgetrennt und der Kolonne vollständig zugeführt. Es wurde weiterhin vom Phasenscheider 135 g/h dem ersten Rührkessel zugeführt. Um die Regeltemperatur in der Kolonne auf 85°C zu halten, wurden zusätzlich der Kolonne 25 g/h organische Phase zugeführt. In der dem Kondensator nachgeschalteten Kühlfalle fielen ca. 5 g/h Flüssigkeit an (ca. 80% IBA, ca. 20% TMA).

Im Sumpf des Fallfilmverdampfers wurde eine wässrige HPA-Lösung von ca. 1,5 kg/h mit ca. 0,4% Isobutyraldehyd und 2,6% HPN ausgeschleust, ca. 69% HPA.

Nach analoger Hydrierung wie in Beispiel 1 wurde eine wässrige Lösung mit ca. 68% NPG, ca. 2,8% HPN, ca. 2,1 Isobutanol, 3,4% Methanol, ca. 2% TMA, Rest Wasser erhalten.

## Patentansprüche

1. Verfahren zur Herstellung von Hydroxypivalinaldehyd durch Aldolisierung von Isobutyraldehyd mit Formaldehyd und anschließender destillativer Aufarbeitung des erhaltenen Reaktionsaustrags, **dadurch gekennzeichnet, dass** der Reaktionsaustrag einer Destillationskolonne zugeführt wird, die bei einem Kopfdruck im Bereich von 0,5 bis 1,5 bar betrieben wird und in der im Kopfbereich eine zweistufige Kondensation vorgesehen ist, bei der die Brüden zunächst in eine bei einer Temperatur im Bereich von 50 bis 80°C betriebenen Par6alkondensator geführt werden, dessen Kondensat zumindest teilweise in die Destillationskolonne zurückgeführt wird, und bei der die im Partialkondensator nicht kondensierten Brüden einem nachgeschalteten, bei einer Temperatur im Bereich von -40 bis +30°C betriebenen Kondensator zugeführt werden, dessen Kondensat zumindest teilweise ausgeschleust wird.

2. Verfahren nach Anspruch 1, **dadurch gekennzeichnet, dass** der Sumpf der Destillationskolonne mit einem Verdampfer mit kurzer Verweilzeit verbunden ist, der bei einer Temperatur im Bereich von 90 bis 130°C betrieben wird.

3. Verfahren nach Anspruch 2, **dadurch gekennzeichnet, dass** der Verdampfer ein Fallfilmverdampfer, Wischfilmverdampfer oder Kurzwegverdampfer ist.

4. Verfahren nach Anspruch 2 oder 3, **dadurch gekennzeichnet, dass** ein mit Hydroxypivalinaldehyd angereichertes Gemisch aus dem Sumpf des Verdampfers ausgeschleust wird.

5. Verfahren nach Anspruch 4, **dadurch gekennzeichnet, dass** das ausgeschleuste Gemisch vor der weiteren Aufarbeitung in einem Kühler mit einer Kühlertemperatur im Bereich von 50 bis 80°C abgekühlt wird.

6. Verfahren nach einem der Ansprüche 1 bis 5, **dadurch gekennzeichnet, dass** die Destillationskolonne Einbauten zur Erhöhung der Trennleistung aufweist und der Reaktionsaustrag der Aldolisierung in einem räumlichen Bereich zwischen %< und 3/4 der theoretischen Böden der Destillationskolonne zugeführt wird.

7. Verfahren nach einem der Ansprüche 1 bis 6, **dadurch gekennzeichnet, dass** das Kondensat des Partialkondensators vollständig in die Kolonne zurückgeführt wird.

8. Verfahren nach einem der Ansprüche 1 bis 7, **dadurch gekennzeichnet, dass** das Kondensat des Partialkondensators in den Kolonnenkopf zurückgeführt wird.

9. Verfahren nach einem der Ansprüche 1 bis 8, **dadurch gekennzeichnet, dass** die Aldolisierung in Gegenwart von Aminen als Base durchgeführt wird.

10. Verfahren zur Herstellung von Neopentylglykol durch Aldolisierung von Isobutyraldehyd mit Formaldehyd und Aufarbeitung des Reaktionsaustrags nach einem Verfahren gemäß einem der Ansprüche 1 bis 9 und nachfolgende Hydrierung des so erhaltenen Hydroxypivalinaldehyds.

## Claims

1. A process for preparing hydroxypivalaldehyde by aldolizing isobutyraldehyde with formaldehyde and subsequently working up the resulting reaction effluent by distillation, which comprises feeding the reaction effluent to a distillation column which is operated at a top pressure in the range from 0.5 to 1.5 bar and in which a two-stage condensation is provided in the top region, in which the vapors are first conducted into a partial condenser operated at a temperature in the range from 50 to 80°C, whose condensate is recycled at least partly into the distillation column, and in which the vapors uncondensed in the partial condenser are fed to a downstream condenser operated at a temperature in the range from -40 to +30°C, whose condensate is at least partly discharged.

2. The process according to claim 1, wherein the bottom of the distillation column is connected to an evaporator which has a short residence time and is operated at a temperature in the range from 90 to 130°C.

3. The process according to claim 2, wherein the evaporator is a falling-film evaporator, wiped-film evaporator or short-path evaporator.

4. The process according to claim 2 or 3, wherein a mixture enriched with hydroxypivalaldehyde is discharged from the bottom of the evaporator.

5. The process according to claim 4, wherein the discharged mixture, before the further workup, is cooled in a condenser with a condenser temperature in the range from 50 to 80 °C.

6. The process according to any of claims 1 to 5, wherein the distillation column has internals for increasing the separating performance and the reaction effluent of the aldolization is fed in a spatial region between ¼ and ¾ of the theoretical plates of the distillation column.

7. The process according to any of claims 1 to 6, wherein the condensate of the partial condenser is recycled fully into the column.

8. The process according to any of claims 1 to 7, wherein the condensate of the partial condenser is recycled into the top of the column.

9. The process according to any of claims 1 to 8, wherein the aldolization is performed in the presence of amines as a base.

10. A process for preparing neopentyl glycol by aldolizing isobutyraldehyde with formaldehyde and working up the reaction effluent by a process according to any of claims 1 to 9 and subsequently hydrogenating the hydroxypivalaldehyde thus obtained.

## Revendications

1. Procédé pour la préparation d'hydroxypivalinaldéhyde par aldolisation d'isobutyraldéhyde avec du formaldéhyde et traitement consécutif par distillation du produit de réaction obtenu, **caractérisé en ce que** le produit de réaction est introduit dans une colonne de distillation qui est exploitée à une pression de tête dans la plage de 0,5 à 1,5 bar et dans laquelle est prévue, dans la zone de tête, une condensation en deux étapes, lors de laquelle les vapeurs sont d'abord guidées dans un condensateur partiel exploité à une température dans la plage de 50 à 80°C, dont le condensat est au moins partiellement recyclé dans la colonne de distillation et lors de laquelle les vapeurs non condensées dans le condensateur partiel sont introduites dans un condensateur disposé en aval, exploité à une température dans la plage de -40 à +30°C, dont le condensat est au moins partiellement évacué.

2. Procédé selon la revendication 1, **caractérisé en ce que** le fond de la colonne de distillation est lié à un évaporateur avec un temps de séjour court, qui est exploité à une température dans la plage de 90 à 130°C.

3. Procédé selon la revendication 2, **caractérisé en ce que** l'évaporateur est un évaporateur à film tombant, un évaporateur à film raclé ou un évaporateur instantané.

4. Procédé selon la revendication 2 ou 3, **caractérisé en ce qu'**un mélange enrichi en hydroxypivalinaldéhyde est soutiré du fond de l'évaporateur.

5. Procédé selon la revendication 4, **caractérisé en ce que** le mélange évacué est refroidi, avant le traitement ultérieur, dans un refroidisseur présentant une température dans la plage de 50 à 80°C.

6. Procédé selon l'une quelconque des revendications 1 à 5, **caractérisé en ce que** la colonne de distillation présente des chicanes pour augmenter la puissance de séparation et le produit de réaction de l'aldolisation est introduit dans une zone spatiale entre 1/4 et 3/4 des plateaux théoriques de la colonne de distillation.

7. Procédé selon l'une quelconque des revendications 1 à 6, **caractérisé en ce que** le condensat du condensateur partiel est recyclé complètement dans la colonne.

8. Procédé selon l'une quelconque des revendications 1 à 7, **caractérisé en ce que** le condensat du condensateur partiel est recyclé dans la tête de colonne.

9. Procédé selon l'une quelconque des revendications 1 à 8, **caractérisé en ce que** l'aldolisation est réalisée en présence d'amines comme base.

10. Procédé pour la préparation de néopentylglycol par aldolisation d'isobutyraldéhyde avec du formaldéhyde et traitement du produit de réaction selon un procédé selon l'une quelconque des revendications 1 à 9 et hydrogénation consécutive de l'hydroxypivalinaldéhyde ainsi obtenu.
